# EUROPEAN PATENT APPLICATION

(11) **EP 2 348 319 A1**
(43) Date of publication of application: **27.07.2011**
(21) Application number: 10290028.9
(22) Date of filing: 21.01.2010
(51) Int. Cl.: G01N 33/96

(54) **Method for adjusting the coagulation time in calibrator or control plasmas**

(71) Applicant: DIAGNOSTICA STAGO (Société par Actions Simplifiée), F-92602 Asnières (FR)
(72) Inventor: Martinoli, Jean-Luc, 92390 Villeneuve la Garenne (FR); Berrendonner, Kristel, 95250 Beauchamp (FR); Barentin, Philippe, 75116 Paris (FR)
(74) Representative: Desaix, Anne

(57) **Abstract**

The invention relates to a method for adjusting the coagulation time during the manufacture of calibrators and controls produced from frozen plasma or plasma treated for lyophilization thereof, for haemostatis tests relating to assaying and to monitoring anticoagulant treatments in a patient.

In the method of the invention, the plasmas selected to prepare the calibrator plasmas or the control plasmas are treated with a view to lyopholization and supplemented with different predefined doses of a drug and a procoagulant factor.

## Description

The invention relates to a method for adjusting the coagulation time during the manufacture of calibrators and controls produced from frozen plasma or plasma treated for lyophilization thereof, for haemostatis tests relating to assaying and to monitoring anticoagulant treatments in a patient.

Haemostatis is considered to be a collection of physiological mechanisms which combine to prevent and stop bleeding. Haemostatis is often compared to a balance, since the fluidity of blood is maintained by dint of an equilibrium between activators and inhibitors of the coagulation phenomenon.

Any disturbance to this equilibrium will tip the balance towards a pathological process: a thrombotic state linked to the formation of a clot in the case of coagulation inhibitor deficiencies or, in contrast, a haemorrhagic state linked to an increase in bleeding in the case of a deficiency of coagulation activating factors.

In outline, the various therapies intended to re-establish haemostatic equilibrium or prevent disturbances or repetitions of disturbances to that equilibrium consist of administering to patients compounds which tend to limit bleeding or, in contrast, of prescribing treatments with an anticoagulant effect depending on whether the risk is haemorrhagic or thrombotic.

Because of the diversity of pathologies in question, of the specificity of the proposed treatments and of the variability of the responses of the patients to such treatments, it may be necessary to carry out regular assays and to regularly monitor the activity of the drugs which are described, using conventional haemostatis tests such as prothrombin time (PT), APTT or other chronometric or chromogenic assays which are routinely carried out in many laboratories. This is particularly the case for patients treated with anti-vitamin K drugs (VKA).

Conventional haemostatis tests are based on measuring the coagulation time of test samples. The measured times are interpreted by comparing the times obtained with reference values which allow the measured times to be correlated to the quantity of drug present in the test sample.

The choice of techniques used generally depends on the type of drug which is to be assayed, and thus the nature of the measured signal could be different depending on the case.

Thus, the measured signal could, for example, be an OD (optical density) or a clot formation time.

In certain cases, such as monitoring a treatment with VKA or heparin, it is necessary to be able to have available calibration plasmas, or calibrator plasmas, in order to obtain the reference values.

These calibrators used for reference values are generally plasmas with a normality which has been verified (*i.e. for which the coagulation times are normal*) to which, for example, known increasing doses of the test drug are added and from which a coagulation time curve is established as a function of the quantity of drug present in the plasma.

Such calibration plasmas as well as control plasmas, if appropriate, may then be present as components in diagnostic kits sold in order to assay certain drugs. Biologists and other consumers of such kits can thus directly determine the quantity of drug present in samples from patients, by transferring the coagulation times for those samples onto the reference curve established using the calibrators, and to verify the proper function of the tests by comparing the values obtained with those of the control plasmas.

However, two problems arise for the manufacturers of *in vitro* diagnostic tests when they have to develop calibration or control plasmas for diagnostic tests the results of which are based on measurements of the coagulation time:
i) the plasma used to prepare the calibrator generally derives from blood transfusion banks and is thus an apheresis plasma. It is thus frozen, but in particular it has been removed over an anticoagulant which may be substantially different from that used in the medical analytical laboratories (sodium citrate, 3.2% - 3.8%). Thus, the citrate concentration may be from 21 to 26 mM/L in a clinical biological laboratory sample, while it is only 12 to 20 mM/L in an apheresis plasma. In the latter case, the anticoagulant may also be accompanied by various adjuvants (dextrose, etc) depending on the apheresis bag manufacturers;
ii) The plasma is defrosted, pooled, supplemented with excipients, filtered and then frozen or lyophilized.

For these reasons, the plasma treated to prepare the calibrator differs substantially from a freshly sampled laboratory sample, particularly as regards the coagulation time. Thus, it is known that the perturbations generated by adjunction of excipients, freezing and lyophilization results in an extension in the prothrombin time (PT) which may be up to 2 or 3 seconds compared with that measured for a fresh plasma.

The skilled person is aware of various ways of at least partially correcting these problems:
- adding buffer substances such as HEPES, which has the effect of restoring the pH of the plasma and thus maintaining the coagulation factor contents (for example: F VIII);
- improving the capacity of the plasma to coagulate by adding activated factor VII (F VIIa).

This second solution has in particular been described in EP-0 706 658.

That document describes a method for preparing a lyophilized calibration plasma obtained from a citrated plasma source in which a predetermined quantity of factor VIIa is added prior to lyophilization.

The quantity of factor VIIa added to the plasma is calculated in order to increase the value of the PT percentage of the (plasma/added factor) association to approximately 100% before lyophilizing the mixture. After reconstitution, such a preparation has PT times which are identical to those measured for normal fresh plasma pools and can thus be used as a calibrator in commercial kits to carry out PT tests.

Another approach which is aimed at correcting the effects of the treatment of plasma and lyophilization on plasma coagulation time consists of re-titrating the lyophilized plasma against a fresh plasma. This is in fact routinely carried out to obtain plasmas which can be used for calibration or monitoring PT when monitoring oral coumarin type anticoagulants (VKA), whether the results are expressed as a percentage or as INR.

Recently, novel oral anticoagulants of the "direct Xa inhibitor" type have been introduced into the market or will soon be introduced.

A priori, such novel drugs do not require regular biological monitoring as with VKAs. Nevertheless, and clearly, there will be situations where an evaluation of the effect will be necessary, for example to identify an overdose or to prove "non-observance".

An example of a compound of this type is rivaroxaban, developed by BAYER laboratories.

Rivaroxaban is an oral direct factor Xa inhibitor indicated for the prevention and treatment of venous thromboembolisms.

Furthermore, a recent publication (SAMAMA et al - poster presented at 20^{th} International Congress on Thrombosis (ICT), Athens, 25-28 June 2008) has stated that assaying of such novel "direct Xa inhibitor" compounds could readily be carried out using PT wherein for which it has been shown that the increase is linearly proportional to the dose of the test drug.

The fact that that technique is universal (and universally employed to monitor VKAs) makes it a good candidate, even more so because it is cheap, which is an important factor as regards public health costs.

However, the same authors also showed that the extension in PT observed with increasing doses of medication differed with the PT reagents employed and that conversion of the measured values into INR did not reduce those variations.

Further, in a recent publication (PERZBORN et al - poster presented at the XXIIth International Society on Thrombosis and Haemostasis (ISTH) - Boston, 11- 16 July 2009), it was shown that oral direct anti-FXa inhibitors could be assayed using chromogenic FXa assays.

The fact that none of the current methods for expressing the results (%, ratio, INR) is readily applicable means that it is necessary to have available calibration plasmas, i.e. normal plasmas overloaded in vitro with the direct factor Xa inhibitor under consideration, in order to develop reliable tests.

Now, surprisingly, the authors of the present invention have experimentally established that when a previously frozen apheresis plasma is overloaded with a direct factor Xa inhibitor, the measured PT extension is disproportional compared with that usually observed when the same manipulation is carried out using a fresh plasma, and that this divergence increases with the quantity of drug added, while the chromogenic activity of FXa on a specific substrate is not modified with respect to that observed for fresh plasmas.

Further, the authors of the present invention have also established that adding an appropriate quantity of a procoagulant factor to treated plasmas supplemented with a drug can correct the observed time divergences compared with fresh plasmas containing the same doses of drug, and that this addition has no influence on the chromogenic activity of FXa on a specific substrate, and thus does not modify the anti-Xa activity of the tested drug.

Thus, the aim of the present invention is to correct the coagulation times obtained for treated plasmas with a view to preparing calibrator plasmas or control plasmas to which an anticoagulant drug has been added before lyophilizing them, in order to retain with such plasmas, following reconstitution, coagulation times which are identical to those obtained for fresh plasmas containing the same doses of drug, without the anti-Xa activity of the drug being modified with respect to that observed for fresh plasmas.

Thus, the present invention provides a method for manufacturing calibrator plasmas or control plasmas for tests for assaying the quantity of drugs having an anticoagulant effect present in the plasma of patients, in which the plasmas selected to prepare the calibrator plasmas or the control plasmas are treated with a view to lyophilization thereof and supplemented with different predefined doses of the drug, **characterized in that** an appropriate dose of a procoagulant factor is added to said plasmas in order to adjust the coagulation time of each plasma, such that following reconstitution, the lyophilized calibrator plasmas or the control plasmas comprising the test drug and the procoagulant factor provide a calibration curve which is identical to that which would be obtained under the same conditions for measurement with fresh plasmas containing the same doses of test drug without adding procoagulant factor.

The present invention also concerns a calibration system for tests for assaying the quantity of an anticoagulant drug present in the plasma of a patient, **characterized in that** it is constituted by samples of lyophilized normal plasmas comprising different pre-defined doses of anticoagulant drug and a procoagulant factor the doses of which are determined such that after reconstitution, said plasmas express coagulation times which are identical to those obtained with fresh plasmas comprising the same doses of drug.

The anticoagulants assayed in accordance with the method of the invention are principally drugs with an "anti-Xa" effect, such as rivaroxaban or apixaban, for example.

The procoagulant factor used is a factor involved in the coagulation cascade, capable of activating the coagulation system. It may be selected to initiate or activate coagulation at any step of the coagulation cascade, especially by activation of a proteolytic enzymatic reaction.

Thus, in a particular implementation, the procoagulant factor may be factor IXa, XIa or XIIa, in the case of an endogenous pathway factor, factor VIIa in the case of an exogenous pathway factor or factor Xa if a common pathway activator is selected.

A procoagulant factor which is preferred in the context of the invention is factor VIIa, preferably used in its recombinant form, which is available from various commercial sources (Novo-Nordisk, American Diagnostica, NIBSC). However, any other component exerting a procoagulant action, tending to reduce the coagulation time of calibration plasmas, may be used in the context of the invention. Thus, factor Xa may be added.

The tests used to measure the coagulation time of the test plasmas are conventional haemostatis tests, such as PT, APTT or another global test which is known to the skilled person. The preferred test which is used is PT.

The coagulation time measurement methods used may be of various natures, such as chronometric methods (clot formation time), chromogenic (measuring the hydrolysis time of a chromogenic substrate), electrochemical or any other type of method which is routinely used for this type of analysis.

Since the calibrator plasmas or control plasmas are lyophilized, excipients are added to the plasma, such as lactose, saccharose, or a HEPES buffer.

The method of the invention is implemented by carrying out the following steps:
i) establishing a coagulation time curve as a function of the dose of anticoagulant drug present in the plasma from normal fresh plasmas or from pools of normal fresh plasmas containing different predetermined doses of said anticoagulant drug;
ii) establishing a coagulation time curve as a function of the dose of anticoagulant drug present in the plasma from normal plasmas or pools of normal plasma containing all of the components necessary for lyophilizaton or freezing and the same doses of anticoagulant drug as for the plasmas of step i);
iii) determining for each value of the coagulation time obtained in step ii) the quantity of procoagulant factor to be added to the plasmas in step ii) to adjust their coagulation time to those obtained with the plasmas of step i) for identical doses of anticoagulant drug, and adding said procoagulant factor to the plasmas of step ii);
iv) lyophilizing or freezing the plasmas from step iii) comprising the predetermined doses of anticoagulant drug and the quantities of procoagulant factor determined in step iii); and, if appropriate:
v) checking the accuracy of the coagulation time adjustment after lyophilization by reconstituting aliquots of plasmas from step iv) and comparing their coagulation times with those of plasmas from step i) respectively comprising the same doses of anticoagulant drug; and optionally
vi) verifying that the anti-Xa activity of the drug in the treated plasmas is not modified with respect to that observed for the plasmas of step i) comprising the same doses of drug.

The plasmas used in step ii) are generally apheresis plasmas originating from transfusion blood banks.

The quantities of procoagulant factor to be added to the calibration and control plasmas are determined as a function of the test drug and of the doses of this drug added to the plasmas.

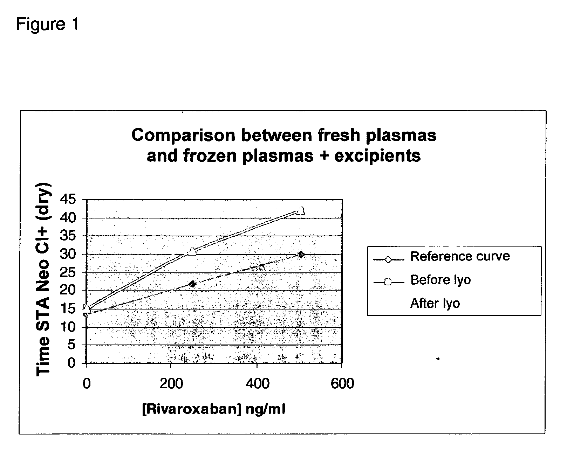

As indicated above, the extension of the coagulation time measured for the plasmas which have been treated to be lyophilized or frozen with respect to the coagulation time of fresh plasmas increases with the doses of the drug added to said plasmas (see Figure 1).

The concentrations of procoagulant factor to add to these calibrators will thus increase with the quantity of drug present in the calibrator.

Calibration is preferably carried out on the basis of 2 to 4 different concentrations of drug.

In accordance with a preferred implementation, the calibration or monitoring system of the invention is obtained using the protocol given below.

A reference curve is produced from a fresh plasma to which predetermined doses of anticoagulant have been added in accordance with step i) of the method of the invention.

The plasma intended to act as a calibrator or control in the context of the present invention is defrosted, pooled, supplemented with lyophilization excipients then filtered.

This plasma is then divided into several batches. Each batch is supplemented with a predetermined quantity of the drug, each quantity being included in a range of concentrations corresponding to the concentrations generally measured in patients, or close to the threshold measurements of said range.

An aliquot portion from each point in the range is taken to produce a range of for procoagulant factor used: for each dose of drug added to the plasma, the procoagulant factor is added to the removed aliquot portions in an increasing quantity determined with respect to the increasing quantities of doses of the drug already added to the plasmas, then a measure of the coagulation time, preferably a PT, is carried out on each sample of the range thus produced.

The quantity of procoagulant factor to be added to obtain the target time (i.e. the PT which would be obtained with a fresh plasma supplemented with the same dose of drug as that present in the test plasma) is determined for each calibration point corresponding to each point in the range or the test control.

In the case of a calibration system intended to assay rivaroxaban, a calibration is preferably carried out using a rivaroxaban concentration range of 0 to 800 ng/L and more preferably 0 to 500 ng/L.

As can be seen from the curve in Figure 1, the PTs carried out on treated plasmas supplemented with different concentrations of rivaroxaban in the range 0 to 500 ng/L show, with respect to fresh plasma supplemented with the same quantities of rivaroxaban, divergences of 0 to 2.4 seconds for 0 ng/L of rivaroxaban added, to 13 seconds for 500 ng/L of rivaroxaban.

In the case in which the procoagulant factor used is factor VIIa, the quantity of this factor to be added to correct this divergence is generally in the range 0.1 to 1 IU/mL, preferably in the range 0.2 to 0.9 IU/mL.

If in the optional step vi) of the method of the invention, it is desired to verify that addition of the procoagulant factor has no influence on the anti-Xa effect of the tested drug, then preferably a test for measuring the enzymatic activity of F Xa on a specific chromogenic substrate is used.

Thus, it is verified that the activity of F Xa is not modified with treated plasmas compared with fresh plasmas comprising identical doses of drug.

As an example, an assay test such as Rotachrom (Diagnostica Stago) may be used; it is an anti-Xa assay test employing competition.

The calibrators and controls obtained after adjusting the coagulation time by adding procoagulant factor are then lyophilized or frozen and may be included in commercial kits intended to monitor anticoagulant treatments in patients.

The present invention thus also concerns a kit for calibration or monitoring in tests for assaying anticoagulant drugs or for monitoring anticoagulant treatments in patients, **characterized in that** it comprises calibrators or controls constituted by lyophilized or frozen plasmas comprising different predetermined doses of the test drug and a procoagulant factor in a quantity sufficient, following reconstitution of said plasmas, to allow an adjustment of their coagulation time to those obtained with fresh reference plasmas comprising identical doses of the drug.

Preferably, the above kit comprises at least two calibrator and/or monitoring plasmas.

In a preferred variation, the kits of the invention are PT calibration or monitoring kits. Their calibrators or controls in this case are advantageously supplemented with F VIIa, preferably in the recombinant form.

The kits of the invention are preferably intended for assaying and monitoring anti-Xa drugs such as rivaroxaban, for example.

The following figures and examples illustrate the invention.
**Figure 1** is an example of a graph showing the increase in the coagulation time divergence (PT in this example) between fresh plasmas and lyophilized plasmas as a function of the anti-Xa dose (rivaroxaban) added to said plasmas;

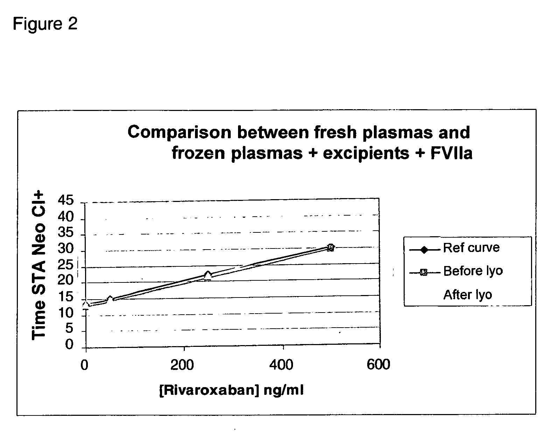

**Figure 2** shows the time adjustment (PT) obtained after addition of factor VIIa to plasmas treated for lyophilization;

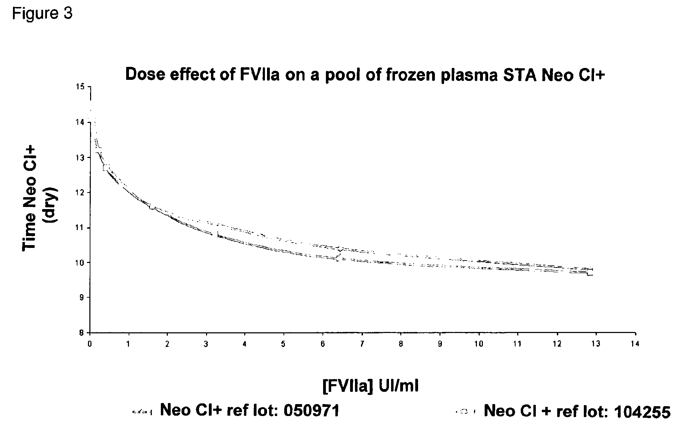

**Figure 3** shows the dose effect of F VIIa with a frozen plasma during NeoCl+ assay;

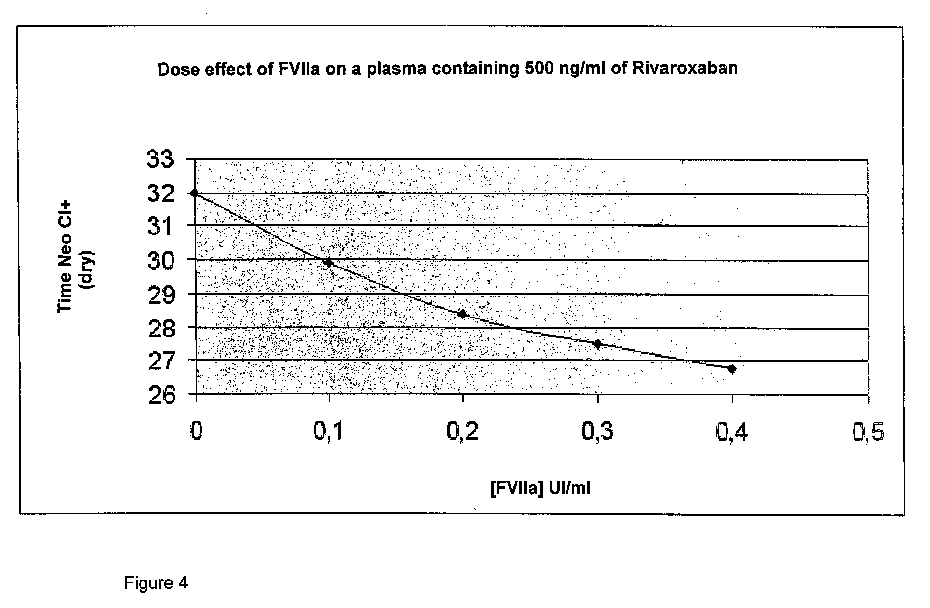

**Figure 4** shows the dose effect of F VIIa on a plasma supplemented with 500 ng/mL of rivaroxaban. The dose of F VIIa which has to be added to the plasma to constitute the calibrator at 500 ng/mL may be determined as a function of the coagulation time to be obtained.

The examples below complete the description of the characteristics of the invention by describing the manufacture of a set of calibrators.

The plasma bags were defrosted at 37°C ± 2°C in a defroster; once defrosted, the plasma was stabilized at 37°C ± 2°C for 30 minutes.

The bags were then pooled in a stainless steel container and the plasma was then homogenized using a magnetic stirrer.

The lyophilization excipients (lactose, saccharose, HEPES buffer) were added, stirring until dissolution was complete.

A sample was taken to measure the pH, the conductivity and to determine the PT on a STA Neo Cl+ apparatus using STA-R (Diagnostica STAGO).

The plasma was filtered on a CRK1 cartridge (Millipore) and a sample was taken to determine the PT in the NeoCl+ after filtration.

The filtered supplemented plasma was separated into four equal parts, then each was supplemented with the appropriate concentration (0 - 500 ng/mL) of rivaroxaban, each plasma supplemented with rivaroxaban constituting a point in the range.

A sample of each point was tested in the STA NeoCl+; a range resulting from the addition of different concentrations of F VIIa was produced for each level as a function of the coagulation times obtained.

For each point, the concentration of added factor VIIa was determined as a function of the times on a reference graph obtained for fresh plasmas.

The F VIIa was then added, and homogenized and a sample of each point was taken in order to measure the pH, the conductivity and to determine the PT in the STA NeoCl+ using STA-R to verify that the desired times were indeed obtained, then each product was distributed and lyophilized.

The table below shows the times obtained in the STA NeoCl+ (PT reagent sold by Diagnostica Stago) at each step of the manufacture.

| *Time in STA Neo CI+ for defrosted plasma pool* | *Time in STA Neo CI+ for plasma pool* + *excipients* | *Time in STA Neo CI+ for plasma pool* + *excipients after filtration* |
|---|---|---|
| ***13.9 sec*** | ***14.4 sec*** | ***14.3 sec*** |

| | *Calibrator A* | *Calibrator B* | *Calibrator C* | *Calibrator D* |
|---|---|---|---|---|
| [Rivaroxaban] theoretical, ng/ml | 0 | 50 | 250 | 500 |
| Time in STA Neo Cl+ after adding rivaroxaban | 14.3 sec | 16.1 sec | 24.6 sec | 35.1 sec |
| Desired time - reference graph | 12.8 sec | 14.4 sec | 20.8 sec | 28.7 sec |
| Time after adding F VIIa before lyophilization | 12.7 sec | 14.4 sec | 21.3 sec | 29.8 sec |
| Time after lyophilization | 13.2 sec | 14.6 sec | 21.7 sec | 33.3 sec |

In the table below, a range for procoagulant factor F VIIa has been produced for a range of concentrations of rivaroxaban.

| | | | | | | |
|---|---|---|---|---|---|---|
| [rivaroxaban] ng/mL [FVIIa] IU/mL | 0 | 0.1 | 0 | 0.2 | 0.3 | |
| Time in NeoCl+, seconds | 13.5 | 12.8 | | 12.4 | 12.2 | |
| [rivaroxaban] ng/mL [FVIIa] IU/mL | 0 | 0.1 | 25 | - | - | |
| Time in NeoCl+, seconds | 14.4 | 13.7 | | - | - | |
| [rivaroxaban] ng/mL [FVIIa] IU/mL | 0 | 0.2 | 50 | 0.3 | 0.4 | |
| Time in NeoCl+, seconds | 15.3 | 14.3 | | 14.6 | 14.6 | |
| [rivaroxaban] ng/mL [FVIIa] IU/mL | 0 | 0.1 | 200 | 0.2 | - | |
| Time in NeoCl+, seconds | 21.2 | 19.8 | | 19 | - | |
| [rivaroxaban] ng/mL [FVIIa] IU/mL | 0 | 0.2 | 250 | 0.3 | 0.4 | |
| Time in NeoCl+, seconds | 23.5 | 21 | | 20.3 | 19.9 | |
| [rivaroxaban] ng/mL [FVIIa] IU/mL | 0 | 0.1 | 500 | 0.2 | 0.3 | 0.4 |
| Time in NeoCl+, seconds | 32 | 29.9 | | 28.4 | 27.5 | 26.8 |
| [rivaroxaban] ng/mL [FVIIa] IU/mL | 0 | 0.2 | 800 | 0.3 | - | |
| Time in NeoCl+, seconds | 43.5 | 38.3 | | 36.6 | - | |

## Claims

1. A method for manufacturing calibrator plasmas or control plasmas for tests for assaying the quantity of drugs having an anticoagulant effect present in the plasma of patients, in which the plasmas selected to prepare the calibrator plasmas or the control plasmas are treated with a view to lyophilization thereof and supplemented with different predefined doses of the drug, **characterized in that** an appropriate dose of a procoagulant factor is added to said plasmas in order to adjust the coagulation time of each plasma, such that following reconstitution, the lyophilized calibrator plasmas or the control plasmas comprising the test drug and the procoagulant factor provide a calibration curve which is identical to that which would be obtained under the same conditions for measurement with fresh plasmas containing the same doses of test drug without adding procoagulant factor.

2. A method according to claim 1, **characterized in that** the assayed drugs with an anticoagulant effect are drugs with an "anti-Xa" effect such as rivaroxaban or apixaban, for example.

3. A method according to claim 1 or claim 2, **characterized in that** the procoagulant factor used is a factor involved in the coagulation cascade, which is capable of activating the coagulation system at the level of an enzymatic coagulation reaction.

4. A method according to any one of the preceding claims, **characterized in that** the procoagulant factor is selected from within the group constituted by: factor IXa, XIa or XIIa in the case of an endogenous pathway activator, factor VIIa in the case of an exogenous pathway activator or factor Xa if a common pathway activator is selected.

5. A method according to any one of the preceding claims, **characterized in that** the procoagulant factor is factor VIIa, in particular is recombinant factor VIIa.

6. A method according to any one of the preceding claims, **characterized in that** the coagulation time is determined by measuring the prothrombin time (PT).

7. A method according to any one of the preceding claims, **characterized in that** it comprises the following steps:
i) establishing a coagulation time curve as a function of the dose of anticoagulant drug present in the plasma from normal fresh plasmas or from pools of normal fresh plasmas containing different predetermined doses of said anticoagulant drug;
ii) establishing a coagulation time curve as a function of the dose of anticoagulant drug present in the plasma from normal plasmas or pools of normal plasma containing all of the components necessary for lyophilizaton or freezing and the same doses of anticoagulant drug as for the plasmas of step i);
iii) determining for each value of the coagulation time obtained in step ii) the quantity of procoagulant factor to be added to the plasmas in step ii) to adjust their coagulation time to those obtained with the plasmas of step i) for identical doses of anticoagulant drug;
iv) lyophilizing or freezing the plasmas from step iii) comprising the predetermined doses of anticoagulant drug and the quantities of procoagulant factor determined in step iii); and, if appropriate:
v) checking the accuracy of the coagulation time adjustment after lyophilization by reconstituting aliquots of plasmas from step iiii) and comparing their coagulation times with those of plasmas from step i) respectively comprising the same doses of anticoagulant drug; and optionally
vi) verifying that the anti-Xa activity of the drug in the treated plasmas is not modified with respect to that observed for the plasmas of step i) comprising the same doses of drug.

8. A method according to claim 7, **characterized in that** the anti-Xa activity of the drug is measured by a test for measuring the enzymatic activity of F Xa on a specific chromogenic substrate.

9. A method according to claim 7, **characterized in that** the plasmas used in step ii) are apheresis plasmas.

10. A calibration and/or monitoring system for tests for assaying the quantity of an anticoagulant drug present in the plasma of a patient, **characterized in that** it is constituted by samples of lyophilized normal plasmas comprising different predefined doses of anticoagulant drug and a procoagulant factor the doses of which are determined such that after reconstitution, said plasmas express coagulation times which are identical to those obtained with normal fresh plasmas or pools of normal fresh plasmas comprising the same doses of the drug.

11. A system for calibration and/or monitoring according to claim 10, **characterized in that** the assayed drugs with an anticoagulant effect are drugs with an "anti-Xa" effect such as rivaroxaban or apixaban, for example.

12. A system for calibration or monitoring according to claim 10 or claim 11, **characterized in that** the procoagulant factor used is a factor involved in the coagulation cascade which is capable of activating the coagulation system.

13. A system for calibration or monitoring according to any one of claims 10 to 12, **characterized in that** the procoagulant factor is factor VIIa, in particular is recombinant factor VIIa.

14. A system for calibration or monitoring according to any one of claims 10 to 13, **characterized in that** the coagulation times are measured using PT.

15. A system for calibration or monitoring according to any one of claims 10 to 14, **characterized in that** the calibration is carried out on the basis of two to four different concentrations of drug.

16. A calibration system according to any one of claims 10 to 15, **characterized in that** the calibration is carried out from a range of concentrations of rivaroxaban ranging from 0 to 800 ng/L, more preferably from 0 to 500 ng/L.

17. A calibration system according to any one of claims 10 to 15, **characterized in that** the quantity of factor VIIa which is added is in the range 0.1 to 1 IU/mL, preferably in the range 0.2 to 0.9 IU/mL.

18. A kit for calibration or monitoring tests for assaying anticoagulant drugs or for monitoring anticoagulant treatments in patients, **characterized in that** it comprises a calibration or monitoring system in accordance with any one of claims 10 to 17.
